(19) Europäisches Patentamt
European Patent Office
Office européen des brevets

(11) Veröffentlichungsnummer: **0 029 176**
**B1**

# (12) EUROPÄISCHE PATENTSCHRIFT

(45) Veröffentlichungstag der Patentschrift: **09.02.83**

(21) Anmeldenummer: **80106813.1**

(22) Anmeldetag: **05.11.80**

(51) Int. Cl.³: **C 07 C 51/56,** C 07 C 53/12, C 07 C 53/122, C 07 C 57/04, C 07 C 63/06, B 01 J 31/00

## (54) Verfahren zur Herstellung von Carbonsäure-Anhydriden.

(30) Priorität: **20.11.79 DE 2946762**

(43) Veröffentlichungstag der Anmeldung: **27.05.81 Patentblatt 81/21**

(45) Bekanntmachung des Hinweises auf die Patenterteilung: **09.02.83 Patentblatt 83/6**

(84) Benannte Vertragsstaaten: **BE DE FR GB IT NL**

(56) Entgegenhaltungen:
**DE-B-1 668 118**
**GB-A-1 044 977**
**ANGEWANDTE CHEMIE, 86. Jahrgang, 1974, Weinheim E. V. DEHMLOW »Phasentransferkatalysierte Zweiphasenreaktionen in der präparativen organischen Chemie« Seiten 187—196**

(73) Patentinhaber: **BASF Aktiengesellschaft, Carl-Bosch-Strasse 38, D-6700 Ludwigshafen (DE)**

(72) Erfinder: **Blatt, Klaus, Dr., Breite Strasse 107, D-6700 Ludwigshafen (DE)**
Erfinder: **Naarmann, Herbert, Dr., Haardtblick 15, D-6719 Wattenheim (DE)**

EP 0 029 176 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Carbonsäure-Anhydriden

Die Erfindung betrifft die Herstellung von Carbonsäureanhydriden aus Carbonsäurechloriden und Carbonsäurealkalisalzen im zweiphasigen System mittels Phasentransferkatalyse.

Die Methoden der Herstellung von Carbonsäureanhydriden mit Acetanhydrid aus dem Säurechlorid mit Pyridin, aus Keten oder Wasserabspaltung sind eingehend beschriebene Verfahren (vgl. Organisch-Chemische Experimentierkunst, Weygand-Hilgetag, J. A. Barth-Verlag, Leipzig 1904, Seiten 458, 460, 375).

Die Umsetzung von Säurehalogeniden in wäßriger Phase mit einer tertiären Base z. B. Pyridin ist ferner in der britischen Patentschrift 1 044 977 beschrieben.

Aus der deutschen Patentschrift ist die Herstellung von gemischten Anhydriden der Sorbinsäure mit Palmitin- oder Stearinsäure allein im organischen Lösungsmittel bekannt, wobei in Kauf genommen wird, daß das Produkt noch Alkalisalze enthält.

Diese Methoden befriedigen für die Herstellung spezieller und reiner Anhydride nicht. Es bestand daher die Aufgabe, ein Verfahren vorzuschlagen, das es erlaubt, in einfacher Weise und in guten Ausbeuten auch sonst schwer herstellbare Anhydride zu gewinnen.

Es wurde nun gefunden, daß es durch Phasentransferkatalyse gelingt, in wäßrigen Systemen mit hoher Ausbeute und in hoher Reinheit — ausgehend vom jeweiligen Carbonsäurechlorid — das Anhydrid herzustellen.

Das Prinzip der Phasentransferkatalyse ist zwar aus dem umfangreichen zusammenfassenden Artikel E. V. Dehmlow, Ang. Chem. 86, (1974) Seiten 187 ff wohlbekannt. Obwohl dort jedoch eine große Zahl von Reaktionen beschrieben ist, u. a. Veresterung und Verätherung, war die Möglichkeit der Herstellung der Anhydride offenbar von den zahlreichen Bearbeitern nicht in Betracht gezogen worden.

In der Tat ist der glatte Verlauf der erfindungsgemäßen Reaktion überraschend.

Demgemäß ist Gegenstand der Erfindung ein Verfahren zur Herstellung von Carbonsäureanhydriden der Formel

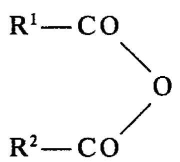

in der $R^1$ und $R^2$ verschiedene oder vorzugsweise gleiche aliphatische oder aromatische Reste bedeuten, wobei man eine Lösung eines Säurechlorids der Formel $R^1-COCl$ in einem mit Wasser nicht mischbaren organischen Lösungsmittel mit einer wäßrigen Lösung von ungefähr äquimolaren Mengen des carbonsauren Salzes der Formel $R^2-COOMe$, in der Me Natrium oder Kalium bedeutet, in Gegenwart eines Phasentransferkatalysators in innige Berührung bringt.

Das gebildete Anhydrid bleibt in der organischen Phase. Die Aufarbeitung erfolgt zweckmäßig durch fraktionierte Destillation und anschließende Reinigung des Anhydrides, ggf. kann aber auch direkt die organische Phase mit dem Anhydrid für Folgereaktionen eingesetzt werden.

Die für die Reaktion geeigneten Phasen-Transfer-Katalysatoren sind z. B. in dem Standardwerk W. P. Weber et al in Phase Transfer Catalyst in Organic Synthesis, Springer Verlag 1977, S. 5/6 beschrieben. Insbesondere kommen Tetracetylammoniumbromid, Tetrabenzylphosphoniumjodid, Tetrabutylammoniumbromid in Betracht. Davon sind die Tetraalkylammoniumhalogenide bevorzugt.

Die angewendete Menge des Phasentransferkatalysators beträgt in der Regel 0,1 bis 15 Gew.-%, vorzugsweise 1 bis 5 Gew.-%, bezogen auf das eingesetzte Säurechlorid.

Das neue Verfahren erlaubt die Herstellung von Säureanhydriden mit hoher Ausbeute und in hoher Reinheit. Durch die Synthese bei Raumtemperatur wird die unerwünschte Bildung störender Nebenprodukte unterdrückt und die organische Phase kann in vorteilhafter Weise sogar direkt weiter verarbeitet werden.

In den nachfolgenden Beispielen bedeuten Teile Gewichtsteile.

Beispiel 1 bis 12

Zu 86 Teilen Methacrylsäure (1 Mol) in 280 Teilen Wasser, 40 Teilen Natriumhydroxid und 10 Teilen Phasentransferkatalysator, werden bei Temperaturen von ca. 10°C, 104,5 Teile Methacrylsäurechlorid (1 Mol) sowie 250 Teile Lösungsmittel zugefügt und 3 Stunden bei Raumtemperatur gerührt. Anschließend wird die organische Phase abgetrennt und durch Destillation aufgearbeitet.

In der nachfolgenden Tabelle sind die Ergebnisse zusammengefaßt.

| Vers. Nr. | Katalysator | Lösungsmittel | Ausbeute % | GC (Reinheit)**) |
|---|---|---|---|---|
| 1 | Tetrabutylammoniumchlorid | Methylenchlorid | 76,9 | 94,2 |
| 2 | Tetrabutylammoniumchlorid | Methylenchlorid | 77,5 | 94,0 |
| 3 | Tetrabutylammoniumchlorid | ohne | 86,4 | 36,2 |
| 4 | N-(Trimethyl-$C_{13}$-$C_{15}$-fettalkyl-ammonium)sulfat | Methylenchlorid | 77,9 | 94,0 |
| 5 | N-(Trimethyl-$C_{13}$-$C_{15}$-fettalkyl-ammonium)sulfat | ohne | 91,6 | 35,7 |
| 6 | Tetrabutylammoniumchlorid | Methylenchlorid | 85,1 | 94,8 |
| 7 | Tetrabutylammoniumchlorid | $CCl_4$ | 85,1 | 85,1 |
| 8 | Tetrabutylammoniumchlorid | $CCl_4$ | 91,1 | 93,4 |
| 9 | Tetrabutylammoniumjodid | $CCl_4$ | 91,6 | 87,1 |
| 10 | Tetrabutylammoniumjodid | Petroläther | 78,1 | 65,6 |
| 11 | Tetrabutylammoniumjodid | Chloroform | 71,4 | 57,5 |

**) GC = Gaschromatographische Reinheitsprüfung.

Beispiel 13 bis 17

Man arbeitet wie in Beispiel 9 beschrieben und ersetzt jeweils das Methacrylsäurechlorid sowie die Methacrylsäure durch 1 Mol eines anderen Säurechlorids bzw. einer anderen Säure und erhält die folgenden Ergebnisse:

| | | | |
|---|---|---|---|
| Nr. 12 | Acrylsäure/chlorid | 89,5 | 92,5 |
| Nr. 13 | Essigsäure/chlorid | 94,3 | 96,7 |
| Nr. 14 | Propionsäure/chlorid | 89,7 | 94,8 |
| Nr. 15 | Benzoesäure/chlorid | 74,2 | 91,7 |

**Patentansprüche**

1. Verfahren zur Herstellung von Carbonsäureanhydriden der Formel

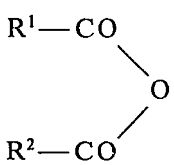

in der $R^1$ und $R^2$ verschiedene oder vorzugsweise gleiche aliphatische oder aromatische Reste bedeuten, dadurch gekennzeichnet, daß man eine Lösung eines Säurechlorids der Formel $R^1-COCl$ in einem mit Wasser nicht mischbaren organischen Lösungsmittel mit einer wäßrigen Lösung von ungefähr äquimolaren Mengen des carbonsauren Salzes der Formel $R^2-COOMe$, in der Me Natrium oder Kalium bedeuten in Gegenwart eines Phasentransferkatalysators in innige Berührung bringt.

2. Verfahren gemäß Anspruch 1, dadurch gekennzeichnet, daß die Reste $R^1$ und $R^2$ niedermolekulare Alkyl- oder Alkenylreste bedeuten.

3. Verfahren gemäß Anspruch 1, dadurch gekennzeichnet, daß die Reste $R^1$ und $R^2$ Isopropenyl

bedeuten.

4. Verfahren gemäß Anspruch 1, dadurch gekennzeichnet, daß man als Phasentransferkatalysatoren 0,1 bis 0,15 Gew.-%, bezogen auf das Säurechlorid eines Tetraalkylammoniumhalogenids verwendet.

## Claims

1. Process for the preparation of carboxylic acid anhydrides of the formula

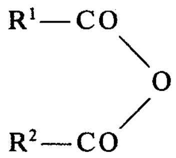

in which $R^1$ and $R^2$ denote different or, preferably, identical aliphatic or aromatic radicals, wherein a solution of an acid chloride of the formula $R^1-COCl$ in a water-immiscible organic solvent is brought into close contact with an aqueous solution of approximately equimolar amounts of the carboxylic acid salt of the formula $R^2-COOMe$, in which Me denotes sodium or potassium, in the presence of a phase transfer catalyst.

2. Process according to claim 1, wherein the radicals $R^1$ and $R^2$ denote low-molecular alkyl or alkenyl radicals.

3. Process according to claim 1, wherein the radicals $R^1$ and $R^2$ denote isopropenyl.

4. Process according to claim 1, wherein 0.1 to 0.15% by weight, based on the acid chloride, of a tetraalkylammonium halide is used as a phase transfer catalyst.

## Revendications

1. Procédé de préparation d'anhydrides d'acides carboxyliques de la formule

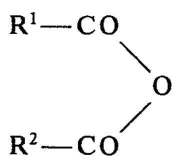

dans laquelle $R^1$ et $R^2$ désignent des restes aliphatiques ou aromatiques différents ou de préférence identiques, caractérisé en ce que l'on mélange intimement, en présence d'un catalyseur de transfert de phases, une solution d'un chlorure d'acide de la formule $R^1-COCl$ dans un solvant organique non miscible à l'eau et une solution aqueuse d'une proportion approximativement équimolaire d'un sel d'acide carboxylique de la formule $R^2-COOMe$, dans laquelle Me désigne le sodium ou le potassium.

2. Procédé suivant la revendication 1, caractérisé en ce que $R^1$ et $R^2$ représentent des radicaux alcoyle ou alcényle à bas poids moléculaire.

3. Procédé suivant la revendication 1, caractérisé en ce que $R^1$ et $R^2$ désignent chacun un radical isopropényle.

4. Procédé suivant la revendication 1, caractérisé en ce que l'on emploie comme catalyseur de transfert de phases un halogénure de tétra-alcoyl-ammonium à raison de 0,1 à 0,15% du poids du chlorure d'acide.